# EUROPEAN PATENT APPLICATION

(11) **EP 1 997 527 A1**
(43) Date of publication of application: **03.12.2008**
(21) Application number: 07716010.9
(22) Date of filing: 24.01.2007
(51) Int. Cl.: A61N 1/36, A61B 5/04

(54) **TRANSCRANIAL ELECTROSTIMULATION DEVICE**

(30) Priority: 23.03.2006 RU 2006109133
(71) Applicant: Lebedev, Valery Pavlovich, St. Petersburg 197022 (RU); Malygin, Aleksandr Vyacheslavovich, Leningradskaya obl. 188680 (RU)
(72) Inventor: Lebedev, Valery Pavlovich, St. Petersburg 197022 (RU); Malygin, Aleksandr Vyacheslavovich, Leningradskaya obl. 188680 (RU)
(74) Representative: Dunleavy, Kevin James
(86) International application number: PCT/RU2007/000027
(87) International publication number: WO 2007/108718

(57) **Abstract**

The device refers to physiotherapy and is intended to protective brain mechanisms stimulation during the treatment of different diseases.

The device of transcranial electrostimulation contains the generator 1, current regulator 4, current stabilizer 3, intensifier 7, current gauge and electrodes 11, which are included to patient condition block 2, timer 5, memory block 6, patient identification block 8 and information image block, and generator, current regulator, current stabilizer 3, intensifier 7, current gauge 9 and electrodes 11 are connected sequentially. The first output of the patient condition block is connected with generator and the second output with the first current input meanwhile, the second input of current regulator is connected to the second intensifier input, the first and second memory blocks are connected with the patient identification block output and procedures counter output 10, the output of which is connected with the current gauge output, and the third input with the memory block output.

## Description

### TECHNICAL FIELD

The invention concerns to physiotherapy and is intended for stimulation of protective mechanisms of brain at the treatment of various diseases.

### BACKGROUND ART

The device for transcranial electrostimulation is known contains a power unit, a current regulator, the current stabilizer, the generator, the amplifier, a current measuring instrument and electrodes. The power unit includes a battery, the power voltage converter, the comparator, the indicator of a battery voltage and a voltage stabilizer. In the device also there is the current stabilizer, the switch and a load equivalent (RU 16826 U1, 2001). The specified device is insufficiently effective and safe at treatment.

### DISCLOSURE OF INVENTION

The invention is directed on development of the safe device of transcranial electrostimulation with high efficiency of treatment of patients, thus the increase of efficiency of the treatment due to introduction of feedback channels with the patient is provided, allowing to arrange parameters of influence depending on a patient's condition, the procedures counter limiting their maximal amount, and also identification means, displaying and storage of the individual information. It allows both to the patient and the doctor to individualize the influence that will lead to the increase of efficiency of treatment and the influence safety.

In the device of transcranial electrostimulations, containing the generator, the current regulator, the current stabilizer, the amplifier, the current measuring instrument and electrodes the patient's condition block, a timer, a memory block, the block of the patient's identification and the information display panel are included in addition, and the generator, the current regulator, the current stabilizer, the amplifier, the current measuring instrument and electrodes are connected sequential. The first output of the patient's condition block is connected to the generator, and its second output is connected to the first input of the current regulator, thus the second input of the current regulator is connected to an output of the memory block, and its third input is connected to the timer output. The current regulator output is connected to the second input of the amplifier, the first and the second inputs of the memory block are connected, correspondingly, to an output of the patient's identification block and an output. The output of the procedures counter is connected also to the timer output, the second output of the procedures counter is connected to the first input of the information display panel the second input of which is connected to an output of the current measuring instrument, and the third input is connected to the memory block output.

The patient's condition block contains the skin resistance gauge, the cardiology signal gauge, the temperature gauge, the biosignals amplifier, ADC and the processor of data processing, and the skin resistant gauge, the cardiology signal gauge and the temperature gauge are connected, correspondingly, to the first, the second and the third inputs of the amplifier of biosignals the output of which through the ADC is connected to the processor of the data processing.

The memory block includes the cells decoder, the block of personal information cells, a socket of external memory modules and the record-reading block, thus and cells the decoder through the block of personal information cells is connected to the first input of the record-reading block, and a socket of external memory modules is connected to the second input of the record-reading block.

### BRIEF DESCRIPTION OF DRAWINGS

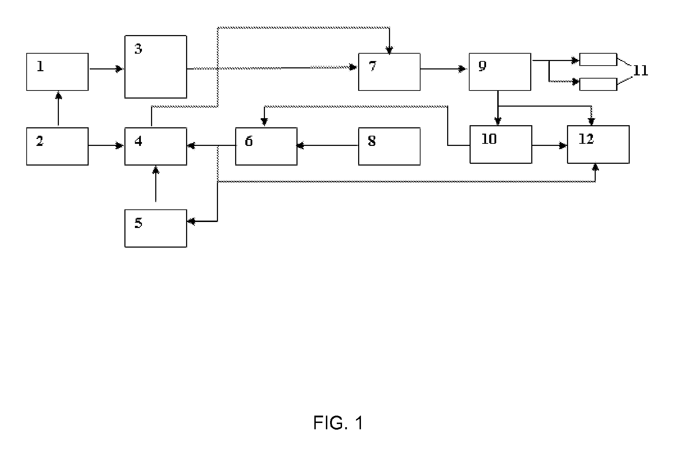

The block diagram of the device is given on the fig. 1.

Fig. 1.1 - the generator, 2 - the patient's condition block, 3 - the current stabilizer, 4 - the current regulator, 5 - the timer, 6 - the memory block, 7 the amplifier, 8 - the block of identification of the patient,

9 - the current measuring instrument, 10 - the procedures counter, 11 - electrodes, 12 - the information display panel.

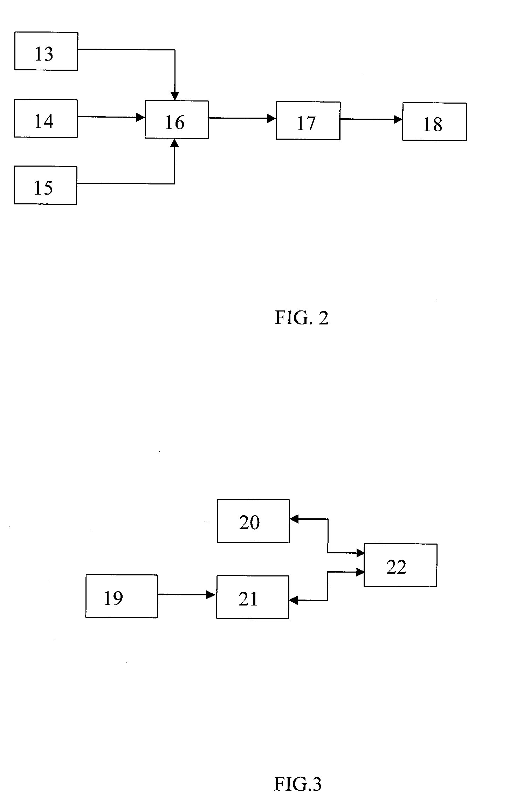

The block diagram of the patient's condition block is represented on the fig. 2.

Fig. 2.13 - the skin resistance gauge, 14 - the cardiology signal gauge, 15 - the temperature gauge, 16 - the amplifier of biosignals, 17 - the ADC , 18 - the processor of data processing.

The block diagram of the memory block is represented on the fig. 3.

Fig. 3.19 - the cells decoder, 20 - a socket of external memory modules, 21 - the block of personal information cells, 22 - the record-reading block.

In the device the pulse generator 1, the current stabilizer 3, the amplifier 7, the current measuring instrument 9 and electrodes 11 are connected sequently. The first output of the patient's condition block 2 is connected to the generator, and the second output is connected to the first input of the current regulator 4, thus the second input of the current regulator 4 is connected to an output of the memory block 6, and the third input is connected to the timer output 5. The output of the current regulator 4 is connected to the second input of the amplifier 7. The first and the second inputs of the memory block 6 are connected, correspondingly, to the output of the block of patient's identification 8 and an output of the procedures counter 10, and its output is connected to the timer input 5. The second output of the procedures counter 10 is connected to the first input of the information display panel 12 the second input of which is connected to the output of the current measuring instrument 9, and the third input is connected to the output of the memory block 6.

The patient's condition block 2 includes the skin resistance gauge 13, the cardiology signal gauge 14 and the temperature gauge 15 which are connected, correspondingly, to the first, the second and the third inputs of the amplifier of biosignals 16, the output of which through the ADC 17 is connected to the processor of data processing 18.

The memory block 6 includes the cells decoder 19, a socket of external memory modules 20 and the block of personal information cells 21 which are connected, correspondingly, to the first and the second inputs of the record-reading block 22.

### BRIEF DESCRIPTION OF THE INVENTION

The device works as follows. After inclusion the device expects the influence on the block of identification of the patient 8. The signal from the output of the block of identification of the patient 8 goes to the first input of the memory block 6 where the personal data concerning the amount of the procedures performed before are read out, and also the data concerning the necessary operating modes for the given patient. The signal from the output of the memory block 6 goes to the second input of the current regulator 4, to the timer input 5 and the third input of the information display panel 12. The signal from the output of the current regulator 4 influences the second input of the amplifier 7, thus determining the intensity of influence. The signal from the timer output5, influencing the third input of the current regulator 4, determines the duration of the procedure. The generator 1 produces the bipolar impulses of current frequency of 72-85 Hz, at duration of a positive impulse 3.7±0.5 ms, duration of a negative impulse 9.2±0.5 ms and a zero average current for the period which go to the input of the current stabilizer 3. Concrete parameters of impulses vary in the set limits during the procedure and depend on the signal which goes to the input of the generator 1 from the first output of the patient's condition block 2. The signal from the second output of the patient's condition's block 2, influencing the first input of the current regulator 4, determines the admissible level of capacity of the device influence. The current impulses from the output of the current stabilizer 3 amplify the amplifier 7 up to the set level and go through the current measuring instrument 9 to the electrodes 11 which are fixed on the patient in a retro mastoidal way. The procedures counter 10 forms a signal of the executed procedure if the current during the procedure exceeded 0.2 MA during at least 15 minutes, and also counts up the amount of the finished procedures for the given patient and the general{ number of the procedures performed with the help of device. The counter forbids the further performance of procedures for one patient at excess of their number within a calendar year more than 50. The information which goes to the inputs of the information display panel 12, is visualized by the later and allows to supervise both the course of current procedure, and personal data of the patient.

The block of the patient's condition 2 works as follows. Signals from the skin resistance gauges 13, cardiology signal gauges 14 and the temperature gauge 15 go to the inputs of the amplifier of biosignals 16 from the output of which after the transformation in the ADC 17 they move to the input of the processor of data processing 18. In the later the data are exposed to the analysis, being based on the known dependences of the influence parameters and on the established limits of norms and pathology. The resulting signal in the form of commands for the current regulator 4 and the generator 1 goes to the block outputs.

The memory block 6 works as follows. Before the beginning of the procedure, being based on the data of the block of the patient's identification 8, the cells decoder 19 produces a signal, allowing to choose one of the cells in the cell block of the personal information 21. The information containing in a personal cell, through the record-reading block 22 goes to the output of the memory block 6. During the procedure the return process occur: the data concerning the patient, going through the record-reading block 22, can be written down in its personal cell. Using a socket of external memory modules 20, in the device through the record-reading block 22 the figures of various types can be entered which are contained on the external media or in computers. For example, the personal data of the patient's group or other device adjustments, etc. Similarly, the data from the device can be transferred to the external media or to the computer.

### INDUSTRIAL APPLICABILITY

In the developed device there are the means of acquisition, storing, imagery and individual information use about the patient that allow to adapt the characteristic influence depending on patient condition. More than that, the introduced procedure counter allows accounting the number of released procedures and limiting their maximal number during the calendar year. So the developed device successfully solves the task of effectiveness increase while increasing the procedures safety.

## Claims

1. the device of transcranial electrostimulation contains the generator, current regulator, current gauge and electrode having the additional patient condition block, timer, memory block, patient identification block and Information panel and generator, current regulator, current stabilizer, intensifier, current gauge and electrode connected sequentially, the first block output of patience condition is connected with generator and the second input with the memory block, the third input with timer output, regulator current output is connected to the second intensifier output, the first and second memory blocks are connected with the patient identification block and procedures block output the output of which is connected to the first panel input of information imagery, the second input of which is connected to the current gauge and the third input with the memory block output.

2. The device according to item 1 differs in the fact that patient condition block contains the cutaneous sensor resistance, cardio signal sensor, temperature sensor, biosignals intensifier, ADS and data processor, and cutaneous sensor resistance, cardio signal and temperature sensor are connected to the first, second and third biosignal intensifiers, the output of which through ADS is connected to data processor.

3. The device according to item 1, 2, differs in fact that memory block includes cell decoder, cell block of the personal information, exterior model memory card socket and recording-reading block through the cell block of personal information is connected to the fist block of recording-reading and external module memory socket to the second block of recording-reading.
